# EUROPEAN PATENT APPLICATION

(11) **EP 4 851 350 A1**
(43) Date of publication of application: **09.12.2026**
(21) Application number: 25748334.7
(22) Date of filing: 16.01.2025
(51) Int. Cl.: D04H 3/009, A61B 5/257, A61F 13/02

(54) **NONWOVEN FABRIC AND USE THEREOF**

(30) Priority: 02.02.2024 JP 2024014758
(71) Applicant: Kuraray Co., Ltd., Kurashiki-shi, Okayama 710-0801 (JP)
(72) Inventor: HOUHASHI, Kimihiko, Saijo-shi, Ehime 793-8585 (JP); SHIRAISHI, Ikuhisa, Saijo-shi, Ehime 793-8585 (JP); OBATA, Soichi, Osaka-shi, Osaka 530-8611 (JP)
(74) Representative: Müller-Boré & Partner Patentanwälte PartG mbB
(86) International application number: PCT/JP2025/001209
(87) International publication number: WO 2025/164336

(57) **Abstract**

Provided is a nonwoven fabric excellent in adhesion to skin and water resistance. The nonwoven fabric includes an elastomer fiber, and has a thickness of 200 µm or less, a ratio (air permeability/basis weight) of an air permeability (cm³/cm²·s) to a basis weight (g/m²) of 1.0 or less, and a bulk density of 0.47 g/cm³ or more. For example, the nonwoven fabric may have the basis weight of 10 to 100 g/m². In the nonwoven fabric, the elastomer fiber may be a urethan-based elastomer fiber.

## Description

### CROSS REFERENCE TO THE RELATED APPLICATION

This application is based on and claims Convention priority to Japanese patent application No. 2024-014758, filed February 2, 2024, the entire disclosure of which is herein incorporated by reference as a part of this application.

### FIELD OF THE INVENTION

The present invention relates to a nonwoven fabric including an elastomer fiber, and a medical patch and a wearable device including the nonwoven fabric.

### BACKGROUND OF THE INVENTION

Conventionally, films have been used as backing materials of medical patches which are applied to skin, such as wound dressings and adhesive plasters. However, the films are low in self-supporting property and likely to be wrinkled, and thus have a problem in handleability.

Therefore, nonwoven fabrics have been proposed as the backing materials of medical patches, and nonwoven fabrics including elastomer fibers have been proposed to ensure conformability when applied to skin. For example, Patent Document 1 (JP Laid-open Patent Publication No. 11-12910) discloses a backing fabric of medical patch, characterized in that: (i) the backing fabric includes an elastic nonwoven fabric in which elastic short fibers composed of an elastomer and having an average diameter of 0.1 to 20 µm, are randomly arranged; (ii) the elastic short fibers are fused to each other at at least one point; and (iii) the elastic nonwoven fabric has a thickness of 0.02 to 1 mm, a water vapor permeability of 500 g/m²·24hr or more, and an air permeability of 90 cc/cm²/sec or less.

Patent Document 2 (JP Laid-open Patent Publication No. 2023-147247) discloses a melt-blown nonwoven fabric including a thermoplastic polyurethane-based elastomer that has a number average molecular weight (Mn) of 100,000 or more and a molecular weight distribution (Mw/Mn) of 2.4 or less, which is a ratio of a weight average molecular weight (Mw) to the number average molecular weight (Mn), and having an average fiber diameter of less than 6.5 µm, and describes an intended use as a medical material.

### CONVENTIONAL ART DOCUMENT

### PATENT DOCUMENT

[Patent Document 1] JP Laid-open Patent Publication No. 11-12910
[Patent Document 2] JP Laid-open Patent Publication No. 2023-147247

### SUMMARY OF THE INVENTION

### PROBLEMS TO BE SOLVED BY THE INVENTION

Since the medical patches need to prevent ingress of moisture and bacteria from the outside, the backing materials of the medical patches are required to have water resistance. While the films are excellent in water resistance, the nonwoven fabrics have voids, causing a problem in water resistance. In fact, the elastic nonwoven fabric actually obtained in Patent Document 1 is inferior in water resistance.

In Patent Document 2, although the water pressure resistance with respect to the basis weight is evaluated, the melt-blown nonwoven fabric actually obtained is insufficient in the water pressure resistance, and there is still room for improvement in the water pressure resistance. Further, the medical patches need to be less likely to be peeled off so that the medical patches can be fixed to skin for a long period of time, and thus require adhesion to the skin, and for the backing materials of the medical patches, thin nonwoven fabrics need to be used in order to reduce the differences in level when applied to the skin. However, the thickness of the melt-blown nonwoven fabric actually obtained in Patent Document 2 is not measured, and the adhesion to skin is unknown.

Therefore, an object of the present invention is to provide a nonwoven fabric excellent in adhesion to skin and water resistance.

### MEANS FOR SOLVING THE PROBLEMS

As a result of intensive studies for solving the above problems, the inventors of the present invention have found that a nonwoven fabric including an elastomer fiber is reduced in thickness to improve the adhesion to skin, while the water resistance has a tendency to be decreased when the thickness is reduced. Then, as a result of further studies, the inventors have found that the water resistance can be improved by reducing the ratio of the air permeability to the basis weight in a specific range and increasing the bulk density in a specific range, leading to the completion of the present invention.

That is, the present invention may include the following aspects.

### [Aspect 1]

A nonwoven fabric including an elastomer fiber, wherein the nonwoven fabric has a thickness of 200 µm or less (preferably 10 to 200 µm, more preferably 13 to 150 µm, further preferably 15 to 100 µm, and even more preferably 20 to 70 µm), a ratio (air permeability/basis weight) of an air permeability (cm³/cm²·s) to a basis weight (g/m²) of 1.0 or less (preferably 0.0005 to 1.0, more preferably 0.0005 to 0.1, further preferably 0.001 to 0.05, and even more preferably 0.003 to 0.01), and a bulk density of 0.47 g/cm³ or more (preferably 0.47 to 1.00 g/cm³, more preferably 0.50 to 0.90 g/cm³, and further preferably 0.53 to 0.80 g/cm³).

### [Aspect 2]

The nonwoven fabric according to aspect 1, having the basis weight of 10 to 100 g/m² (preferably 15 to 80 g/m², more preferably 20 to 60 g/m², and further preferably 21 to 45 g/m²).

### [Aspect 3]

The nonwoven fabric according to aspect 1 or 2, wherein the elastomer fiber is a urethane-based elastomer fiber.

### [Aspect 4]

The nonwoven fabric according to any one of aspects 1 to 3, having a stress at 25% elongation of 10 N/5 cm or less (preferably 8 N/5 cm or less, and more preferably 7 N/cm or less) in at least one direction in a plane direction.

### [Aspect 5]

The nonwoven fabric according to any one of aspects 1 to 4, having an elongation of 200% or more (preferably 250% or more, and more preferably 280% or more) in at least one direction in a plane direction.

### [Aspect 6]

The nonwoven fabric according to any one of aspects 1 to 5, having a water pressure resistance of 170 mmH₂O or more (preferably 200 mmH₂O or more, and more preferably 230 mmH₂O or more) measured in accordance with a low water pressure method in JIS L 1092:2009.

### [Aspect 7]

The nonwoven fabric according to any one of aspects 1 to 6, wherein the nonwoven fabric is a layered body including nonwoven fabrics that are different in average fiber diameter.

### [Aspect 8]

A medical patch including the nonwoven fabric according to any one of aspects 1 to 7.

### [Aspect 9]

The medical patch according to aspect 8, including a pressure-sensitive adhesive layer.

### [Aspect 10]

A wearable device including the nonwoven fabric according to any one of aspects 1 to 7.

### [Aspect 11]

The wearable device according to aspect 10, including a pressure-sensitive adhesive layer.

As used in the present specification, the singular forms "a", "an", and "the" are intended to include the plural forms including "at least one", unless the content clearly indicates otherwise. As used in the present specification, the terms "and/or", "at least one", and "one or more" include any and all combinations of the relevant listed items.

Any combination of at least two constructions, disclosed in the appended claims and/or the specification and/or the accompanying drawing should be construed as included within the scope of the present invention. In particular, any combination of two or more of the appended claims should be equally construed as included within the scope of the present invention.

### EFFECT OF THE INVENTION

The nonwoven fabric according to the present invention is excellent in adhesion to skin and water resistance. The nonwoven fabric according to the present invention can be used for medical patches and wearable devices.

### BRIEF DESCRIPTION OF THE DRAWINGS

The present invention will become more clearly understood from the following description of a preferred embodiment thereof, when taken in conjunction with the accompanying drawing. The drawings are not necessarily shown at a consistent scale and are exaggerated in order to illustrate the principle of the present invention. However, the embodiment and the drawing are given only for the purpose of illustration and explanation, and are not to be taken as limiting the scope of the present invention in any way whatsoever, which scope is to be determined by the appended claims.
[Fig. 1A] Fig. 1A is a schematic perspective view of a nonwoven fabric according to one embodiment for illustrating a method for measuring a bending resistance.
[Fig. 1B] Fig. 1B is a schematic perspective view of a nonwoven fabric according to one embodiment for illustrating a method for measuring a bending resistance.
[Fig. 1C] Fig. 1C is a schematic perspective view of a nonwoven fabric according to one embodiment for illustrating a method for measuring a bending resistance.
[Fig. 2] Fig. 2 is a schematic cross-sectional view showing one embodiment of a collection method in a melt-blowing method.
[Fig. 3] Fig. 3 is a schematic cross-sectional view showing one embodiment of a collection method in a melt-blowing method.
[Fig. 4] Fig. 4 is a schematic cross-sectional view showing one embodiment of a collection method in a melt-blowing method.

### DESCRIPTION OF EMBODIMENTS

A nonwoven fabric includes an elastomeric fiber. Since the elastomer fiber has excellent stretchability and low tensile stress, incorporation of the elastomer fiber can improve stretchability and flexibility of the nonwoven fabric, and thus can improve conformability when the nonwoven fabric is applied to skin.

The elastomer fiber is a fiber containing an elastomer, and the elastomer may be preferably a thermoplastic elastomer. The thermoplastic elastomer is a copolymer containing a hard segment and a soft segment, and specific examples thereof include urethane-based elastomers, styrene-based elastomers, olefin-based elastomers, ester-based elastomers, amide-based elastomers, and vinyl chloride-based elastomers depending on the type of the hard segment. The elastomer fiber may be preferably a urethane-based elastomer fiber containing a urethane-based elastomer, a styrene-based elastomer fiber containing a styrene-based elastomer, and an ester-based elastomer fiber containing an ester-based elastomer from the viewpoint of thermal stability at the time of manufacturing the nonwoven fabric, and may be more preferably the urethane-based elastomer fiber from the viewpoint of excellent stretchability. The nonwoven fabric may include these elastomer fibers singly, or in combination of two or more thereof.

The elastomer fiber may contain other components than the elastomer as long as the effects of the present invention are not impaired. Examples of the other components include thermoplastic resins and various additives. Examples of the thermoplastic resins include polyolefin-based resins such as a polyethylene, a polypropylene, a polybutene, a polymethylpentene, and an ethylene-propylene copolymer; polyester-based resins such as a polyethylene terephthalate, a polybutylene terephthalate, and a polyethylene naphthalate; polyamide-based resins such as a polyamide 6 and a polyamide 66; and acrylic polymers such as a polyacrylic acid, a polymethacrylic acid, a polyacrylic acid ester, and a polymethacrylic acid ester. The elastomer fiber may contain the elastomer in an amount of 50 wt% or more, preferably 60 wt% or more, more preferably 80 wt% or more, further preferably 90 wt% or more, and even more preferably 95 wt% or more.

From the viewpoint of enhancing the water resistance of the nonwoven fabric, it is preferable to improve hydrophobicity of the nonwoven fabric, and for example, a hydrophobic group may be introduced into a molecule of the elastomer contained in the elastomer fiber. For example, a hydrophobic group such as a hydrocarbon group or a fluorine-containing group may be introduced into a molecular end of the elastomer, and in the case of the hydrocarbon group, hydrophobicity can be adjusted by the carbon number of a carbon chain. Further, the hydrophobic group may be introduced into a molecular chain of the elastomer, and for example, the urethane-based elastomer contains a soft segment formed from a long-chain polyol and a diisocyanate and a hard segment formed from a short-chain glycol and a diisocyanate, and a hydrophobic polyol such as a polybutadiene polyol and an acrylic polyol may be used as the polyol of the soft segment.

The elastomer fibers constituting the nonwoven fabric may have an average fiber diameter of 20 µm or less from the viewpoint of enhancing the denseness of the nonwoven fabric. From the viewpoints of enhancing the denseness of the nonwoven fabric and improving the self-supporting property thereof, the average fiber diameter of the elastomer fibers may be preferably 0.1 to 18 µm, and more preferably 1 to 15 µm. In the present specification, when a cross section (fiber cross section) orthogonal to a fiber axis direction of the elastomer fiber is not a perfect circle (for example, a modified cross section such as an oval and a polygon), a cross-sectional area of the fiber cross section is measured, and on the basis of assumption that the fiber cross section is a perfect circle, the diameter of the perfect circle having the cross-sectional area is converted as a fiber diameter.

The elastomer fibers constituting the nonwoven fabric may be long fibers (filaments) or short fibers (staples), and may be preferably the long fibers from the viewpoint of stretchability. In the present specification, the long fiber refers to a fiber having a certain continuous length, and can be distinguished from the short fiber intentionally cut into a predetermined fiber length. For example, a fiber constituting a filament nonwoven fabric obtained by a melt-blowing method or a spunbonding method is regarded as the long fiber.

The nonwoven fabric may include an elastomer fiber whose fiber cross section is a modified cross section. In the present specification, the modified cross section means a fiber cross section in a shape that is not a perfect circle. When a nonwoven fabric is obtained by pressing a precursor nonwoven fabric as in the method for producing the nonwoven fabric as described later, even if the fiber cross section of the elastomer fiber constituting the precursor nonwoven fabric has a perfect circular shape, the elastomer fiber is compressed by the pressing, thereby deforming the fiber cross section from the perfect circular shape to a modified cross section.

The nonwoven fabric may contain the elastomer fiber in an amount of 50 wt% or more, preferably 80 wt% or more, more preferably 90 wt% or more, further preferably 95 wt% or more, and even more preferably 98 wt% or more.

From the viewpoint of enhancing the water resistance of the nonwoven fabric, the nonwoven fabric may further contain hydrophobic fibers besides the elastomer fibers. Examples of the hydrophobic fibers include polyolefin-based fibers such as polyethylene fibers and polypropylene fibers, and polyester-based fibers.

From the viewpoint of enhancing the water resistance of the nonwoven fabric, a water repellent may be provided to the surface of the fiber constituting the nonwoven fabric, or a water repellent may be mixed as an additive in the fiber. Examples of the water repellent include a fluorine-based water repellent, a polysiloxane-based water repellent, and a paraffin-based water repellent.

The nonwoven fabric has a thickness of 200 µm or less. In the case of using the nonwoven fabric as a backing material for a medical patch or the like, which is applied to skin for use, the adhesion to the skin can be improved by reducing the thickness of the nonwoven fabric. Specifically, when the patch is applied to the skin, the difference in level between the skin and the patch can be reduced, thus allowing the reduction of catching on something outside, and allowing the edge to be prevented from being peeled off, and as a result, the patch can be made less likely to be peeled off. In addition, the nonwoven fabric is well conformability to the skin, thus allowing discomfort to be reduced when applied to the skin. The thickness of the nonwoven fabric may be preferably 150 µm or less, more preferably 100 µm or less, and further preferably 70 µm or less. On the other hand, the lower limit of the thickness of the nonwoven fabric is not particularly limited to a specific one, and may be, for example, 10 µm or more, preferably 13 µm or more, more preferably 15 µm or more, and further preferably 20 µm or more from the viewpoint of self-supporting property. In the present specification, the thickness is a value measured by the method described in Examples below.

The nonwoven fabric has a ratio (air permeability/basis weight) of an air permeability (cm³/cm²·s) to a basis weight (g/m²) of 1.0 or less, and a bulk density of 0.47 g/cm³ or more. Even when the thickness of the nonwoven fabric is small as described above, the water resistance can be improved by reducing the ratio of the air permeability to the basis weight in a specific range and increasing the bulk density in a specific range. The bulk density is an index indicating how much the nonwoven fabric is three-dimensionally packed with fibers, and indicates that the higher the bulk density is, the fewer the void is and the more densely the nonwoven fabric is packed with the fibers. Further, the air permeability is an index indicating the degree of unevenness in fiber density. More specifically, the air permeability is determined by measuring the amount of air passing through the gaps between the fibers of the nonwoven fabric, and increased unevenness in fiber density increases the amount of air passing from areas with fewer fibers, so that such a nonwoven fabric has a tendency to have higher air permeability than the nonwoven fabric in which the unevenness in fiber density is reduced. The air permeability is, as a ratio (air permeability/basis weight) to the basis weight, expressed by an index considering the amount of fibers, which represents that the less the ratio of the air permeability to the basis weight is, the less the unevenness in fiber density is. In general, since a nonwoven fabric includes fibers, voids are between the fibers and water penetrates from the voids, and it is thus difficult to improve the water resistance. However, where both the ratio of the air permeability to the basis weight and the bulk density are in specific ranges, the nonwoven fabric has a structure densely packed with the fibers without unevenness, and thus, water pressure for penetration into the nonwoven fabric can be suppressed, and the nonwoven fabric is excellent in the water resistance.

The nonwoven fabric may have the ratio (air permeability/basis weight) of the air permeability to the basis weight of preferably 0.1 or less, more preferably 0.05 or less, and further preferably 0.01 or less. Further, the lower limit of the ratio (air permeability/basis weight) of the air permeability of the nonwoven fabric to the basis weight thereof is not particularly limited to a specific one, and may be, for example, 0.0005 or more, preferably 0.001 or more, and more preferably 0.003 or more from the viewpoint of improving moisture permeability.

The nonwoven fabric may have the air permeability of 10 cm³/cm²·s or less, preferably 3.0 cm³/cm²·s or less, more preferably 1.0 cm³/cm²·s or less, and further preferably 0.8 cm³/cm²·s or less from the viewpoint of reducing the unevenness in fiber density. Further, the lower limit of the air permeability of the nonwoven fabric is not particularly limited to a specific one, and may be, for example, 0.1 cm³/cm²·s or more from the viewpoint of improving moisture permeability. In the present specification, the air permeability is a value measured by the method described in Examples below.

The nonwoven fabric may have the basis weight of 10 to 100 g/m², preferably 15 to 80 g/m², more preferably 20 to 60 g/m², and further preferably 21 to 45 g/m² from the viewpoint of self-supporting property and flexibility. In the present specification, the basis weight is a value measured by the method described in Examples below.

The nonwoven fabric may have the bulk density of preferably 0.50 g/cm³ or more, and more preferably 0.53 g/cm³ or more. The upper limit of the bulk density of the nonwoven fabric is not particularly limited to a specific one, and may be, for example, 1.00 g/cm³ or less, preferably 0.90 g/cm³ or less, and more preferably 0.80 g/cm³ or less from the viewpoint of moisture permeability and flexibility. In the present specification, the bulk density is a value measured by the method described in Examples below.

The nonwoven fabric may have the thickness, the ratio of the air permeability to the basis weight, and the bulk density in the specific ranges described above, and may be a layered body including a plurality of nonwoven fabric layers. In the present specification, even when the nonwoven fabric is a layered body, the thickness, the ratio of the air permeability to the basis weight, and the bulk density are considered as measured values for the whole layered body. By layering a plurality of nonwoven fabrics, the properties, such as the water resistance and the moisture permeability, of the layered body can be adjusted depending on differences in the embodiments of the nonwoven fabrics, and for example, the water resistance can be enhanced while ensuring the moisture permeability. For the layered body, the nonwoven fabrics may be directly layered with each other, or may be layered with each other with an adhesive interposed therebetween as long as the effects of the present invention are not impaired. The layered body may include nonwoven fabrics that are different in average fiber diameter. For example, the average fiber diameter of the nonwoven fabric with the minimum average fiber diameter in the layered body may be 0.1 to 15 µm, and preferably 1 to 13 µm. The average fiber diameter ratio (maximum/minimum) of the nonwoven fabric with the maximum average fiber diameter to the nonwoven fabric with the minimum average fiber diameter in the layered body may be 1.1 to 20, preferably 1.1 to 10, and more preferably 1.1 to 5.0. The average fiber diameter ratio in the above range makes the fiber shapes of fibers with larger average fiber diameters likely to remain when the layered body is produced by pressing, and when the nonwoven fabric layer with a larger average fiber diameter is used on the surface side opposite to skin, catching on something outside can be reduced, and the layered body can be thus made less likely to be peeled off.

From the viewpoints of flexibility and conformability to skin, the nonwoven fabric may have a stress at 25% elongation of 10 N/5 cm or less, preferably 8 N/5 cm or less, and more preferably 7 N/cm or less in at least one direction in a plane direction. Further, the lower limit of the stress at 25% elongation of the nonwoven fabric is not particularly limited to a specific one, and may be, for example, 1 N/5 cm or more, preferably 2 N/5 cm or more, and more preferably 3 N/5 cm or more from the viewpoint of self-supporting property. The above range of the stress at 25% elongation is preferably satisfied in two directions (for example, MD and CD) orthogonal to each other in the plane direction. In the present specification, the stress at 25% elongation is a value measured by the method described in Examples below.

From the viewpoint of stretchability and conformability to skin, the nonwoven fabric may have an elongation of 200% or more, preferably 250% or more, and more preferably 280% or more in at least one direction in a plane direction. The upper limit of the elongation of the nonwoven fabric is not particularly limited to a specific one, and may be, for example, 600% or less, preferably 500% or less, and more preferably 400% or less. The above range of the elongation is preferably satisfied in two directions (for example, MD and CD) orthogonal to each other in the plane direction. In the present specification, the elongation means an elongation at break in a tensile test, and is a value measured by the method described in Examples below.

The nonwoven fabric is excellent in the water resistance, and may have a water pressure resistance of 170 mmH₂O or more, preferably 200 mmH₂O or more, more preferably 230 mmH₂O or more, and further preferably 280 mmH₂O or more, measured in accordance with a low water pressure method in JIS L 1092:2009. The upper limit of the water pressure resistance of the nonwoven fabric is not particularly limited to a specific one, and may be, for example, 1500 mmH₂O or less.

The nonwoven fabric preferably has rigidity from the viewpoint of excellence in self-supporting property, and may have, for example, a bending resistance of 5 mm or more. In the present specification, the bending resistance is measured as follows: preparing a nonwoven fabric sample having a long side of 10 cm and a short side of 2 cm, in which the long side is along an MD (machine direction; a flow direction at the time of manufacturing the nonwoven fabric) of the nonwoven fabric and the short side is along a CD (cross direction; a direction orthogonal to the MD) thereof; placing the nonwoven fabric sample on a horizontal surface; folding the nonwoven fabric sample by lifting up one short side end and overlaying it on the other short side end; pressing the bent part (substantially central part in the long side direction) with a load of 19 kg/m² for 1 second; and then observing a curved surface shaped part of the nonwoven fabric sample, which is formed when the load is removed, from a side surface of the nonwoven fabric sample, to measure a distance from the horizontal surface to the maximum height of the curved surface shaped part as the bending resistance. The more the bending resistance is, the stiffer the nonwoven fabric is, the more excellent self-supporting property is, and the more excellent in handleability. Further, the upper limit of the bending resistance is not particularly limited to a specific one, and may be, for example, 30 mm or less from the viewpoint of flexibility.

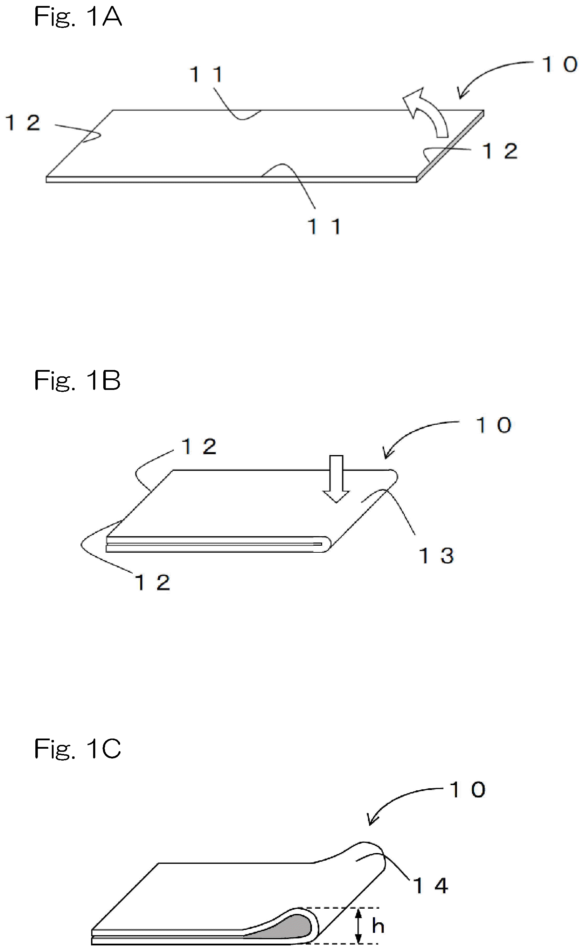

The method for measuring the bending resistance will be described in more detail with reference to schematic perspective views of the nonwoven fabric in Figs. 1A, 1B, and 1C. Fig. 1A shows a nonwoven fabric 10 that has long side ends 11 and short side ends 12. The nonwoven fabric 10 is placed on a horizontal surface, and one short side end 12 is lifted up as indicated by an arrow in Fig. 1A and folded so as to be overlaid on the other short side end 12. Fig. 1B shows the nonwoven fabric 10 that is folded so that one short side end 12 is overlaid on the other short side end 12. Then, a bent part 13 formed at the substantially central part in the long side direction when folded is pressed by applying a load thereto as indicated by an arrow in Fig. 1B. Thereafter, when the load is removed, the bent part 13 is restored on the basis of rigidity of the nonwoven fabric 10 to form a curved surface shaped part 14. Fig. 1C shows the nonwoven fabric 10 with the curved surface shaped part 14 formed. Then, a distance h in the vertical direction from the horizontal surface to the maximum height of the curved surface shaped part 14 formed by the nonwoven fabric 10 can be measured as the bending resistance.

A method for producing the nonwoven fabric may include: preparing a precursor nonwoven fabric; and pressing the precursor nonwoven fabric. Pressing the precursor nonwoven fabric can, depending on the pressing conditions, reduce the thickness, and compress the precursor nonwoven fabric so as to increase the fiber denseness, thereby reducing the air permeability and increasing the bulk density. In order to produce the nonwoven fabric, adjusting a basis weight, a thickness, a fiber distribution, a fiber orientation, and the like of the precursor nonwoven fabric allows adjustments to achieve desired thickness, air permeability, and bulk density.

A structure of the precursor nonwoven fabric is not particularly limited to a specific one as long as the nonwoven fabric can be produced, and the precursor nonwoven fabric may be a wet-laid nonwoven fabric, a dry-laid nonwoven fabric, a direct-spun nonwoven fabric (such as a spunbonded nonwoven fabric and a melt-blown nonwoven fabric), or the like. The precursor nonwoven fabric may preferably be a melt-blown nonwoven fabric from the viewpoint of allowing the voids between fibers to be reduced and allowing the denseness to be enhanced by subsequent pressing.

The melt-blown nonwoven fabric can be formed by discharging a molten elastomer from a spinneret, directly blowing a heated high-speed air flow from air nozzles incorporated in the spinneret, pulling out fibrous discharged materials together with a high-temperature and high-speed air flow from the spinneret, and collecting the fibrous discharged materials on a collection surface. In the melt-blowing method, the fiber diameter can be adjusted by conditions such as a hole diameter of the spinneret, discharge amount and a collection distance from the spinneret to the collection surface, the basis weight and the thickness can be adjusted by conditions such as a moving speed (collection speed) of the collection surface, and the basis weight and thickness of the precursor nonwoven fabric may be adjusted from the viewpoint of achieving desired thickness, air permeability, and bulk density by the pressing described later. For example, the collection speed may be 5 to 30 m/min, preferably 8 to 25 m/min, and more preferably 10 to 20 m/min, although the collection speed varies depending on the fiber diameter of the fibers to be collected.

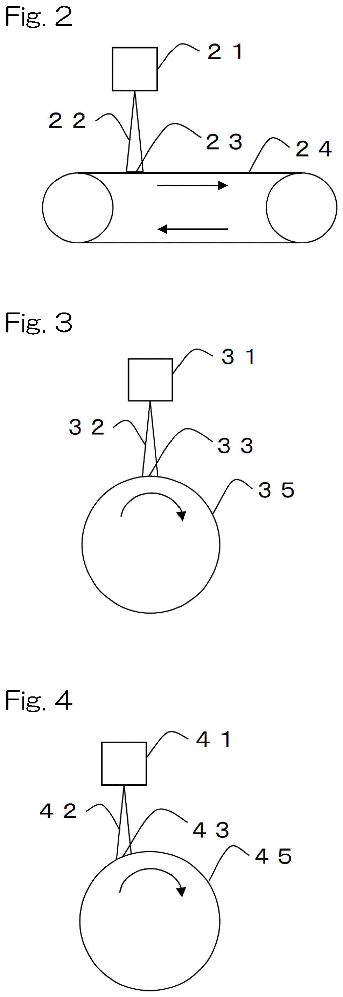

The melt-blown nonwoven fabric can be continuously formed in the machine direction (MD) by collecting the fibrous discharged materials from the spinneret on the moving collection surface. For the collection surface, a perforated collection surface such as a net may be used, and the collection surface may have a suction for sucking air. The suction can suck an air flow blown from the spinneret and promote the collection on the collection surface. The collection method may be a method of using a conveyor belt for collecting on a planar surface as shown in Fig. 2, or a method of using a rotating roll for collecting on a curved surface as shown in Figs. 3 and 4.

Fig. 2 is a schematic cross-sectional view showing a method of collecting on a planar surface as a collection method in a melt-blowing method. In Fig. 2, fibrous discharged materials 22 discharged together with a high-temperature and high-speed air flow from a spinneret 21 are discharged onto a collection surface 23 in a conveyor belt 24 and collected on a planar surface.

Figs. 3 and 4 are schematic cross-sectional views each showing a method of collecting on a curved surface as a collection method in a melt-blowing method. In Fig. 3, fibrous discharged materials 32 discharged together with a high-temperature and high-speed air flow from a spinneret 31 are discharged onto a collection surface 33 in a rotating roll 35 and collected on a curved surface. The collection surface 33 is an outer peripheral surface of the rotating roll 35 substantially perpendicular to the discharge direction from the spinneret 31.

In Fig. 4, fibrous discharged materials 42 discharged together with a high-temperature and high-speed air flow from a spinneret 41 are discharged onto a collection surface 43 in a rotating roll 45 and collected on a curved surface. The collection surface 43 is an outer peripheral surface located upstream of the collection surface 33 in Fig. 3 in the rotating direction of the rotating roll 45, and is an outer peripheral surface of the rotating roll 45 inclined with respect to the discharge direction from the spinneret 41. The collection surface in the rotating roll can be set depending on the direction of the spinneret and the positional relationship between the spinneret and the rotating roll.

As for the collection method, from the viewpoint of adjusting the distribution and orientation of fibers, the materials are preferably collected on a curved collection surface with a suction roll. Using a curved surface such as the outer peripheral surface of the roll as the collection surface allows the air flow blown from the spinneret to flow along the curved surface shape, and accordingly, collected fibers can be made more likely to be oriented and evenly distributed. Orienting and more uniformly distributing the fibers of the precursor nonwoven fabric allow the fiber denseness to be enhanced by the pressing described later, thereby allowing the air permeability and the bulk density of the nonwoven fabric to be adjusted. Further, by positioning the collection surface on the outer peripheral surface on the upstream side in the rotating direction of the rotating roll as shown in Fig. 4, the air flow blown from the spinneret can be made less likely to flow in the machine direction (the rotating direction of the rotating roll) to reduce the amount of air flowing in the machine direction. As a result, probably since the nonwoven fabric formed by collecting the fibers can be prevented from flapping on the collection surface, the denseness of the structure of the fibers can be enhanced. In addition, probably since the nonwoven fabric can be further prevented from flapping on the collection surface by sucking air with the suction roll, the denseness of the structure of the fibers can be further enhanced. Therefore, the precursor nonwoven fabric obtained at the collection position in Fig. 4 can reduce the air permeability and increase the bulk density after being subjected to the pressing described later, as compared with the precursor nonwoven fabric obtained at the collection position in Fig. 3.

The precursor nonwoven fabric may have a basis weight of 10 to 100 g/m², preferably 15 to 80 g/m², more preferably 20 to 60 g/m², and further preferably 21 to 45 g/m² from the viewpoint of adjusting the air permeability and the bulk density of the nonwoven fabric subjected to the pressing.

The precursor nonwoven fabric may have a thickness of 20 to 400 µm, preferably 30 to 300 µm, more preferably 40 to 200 µm, and further preferably 50 to 100 µm from the viewpoint of adjusting the thickness and the bulk density of the nonwoven fabric subjected to the pressing.

The elastomer fibers constituting the precursor nonwoven fabric may have an average fiber diameter of 20 µm or less, preferably 0.1 to 18 µm, and more preferably 1 to 15 µm from the viewpoint of enhancing the denseness of the nonwoven fabric subjected to the pressing.

The precursor nonwoven fabric may be a precursor layered body in which a plurality of nonwoven fabrics are layered. For the precursor layered body, a plurality of nonwoven fabrics may be simply overlaid, and the nonwoven fabrics may be integrated by the pressing described later. For the precursor layered body, a plurality of melt-blown nonwoven fabrics may be preferably layered, and in such a case, the precursor layered body may be produced by separately preparing and overlaying the melt-blown nonwoven fabrics, or by using a melt-blown nonwoven fabric as a support, and forming another melt-blown nonwoven fabric by directly spinning thereon to obtain a layered body.

Then, the precursor nonwoven fabric may be subjected to pressing to compress the precursor nonwoven fabric so as to reduce the thickness and increase the fiber denseness, thereby reducing the air permeability and increasing the bulk density. The pressing can be appropriately adjusted depending on the structure of the precursor nonwoven fabric, and may be preferably continuously performed, for example, by calendering in which heat treatment is performed by compressing with heated rolls. Hot pressing allows the elastomer fibers to be easily deformed, and allows a densely packed structure to be formed. In addition, the elastomer fibers can be fused to each other to adjust the air permeability and transparency.

In the case of calendering, a linear pressure between a pair of metal rolls or between a rubber roll and a metal roll can be appropriately set depending on the thickness, the basis weight, and the like of the precursor nonwoven fabric, and may be, for example, 20 to 80 kg/cm, preferably 30 to 70 kg/cm, and more preferably 35 to 60 kg/cm.

A heating temperature in the calendaring, which also depends on the linear pressure between the rolls, can be appropriately set depending on the type of the elastomer fiber constituting the precursor nonwoven fabric, in order to adjust the degree of fusion between the elastomer fibers. In the case of a urethane-based elastomer fiber, for example, the heating temperature for the metal roll may be 100 to 150°C, preferably 110 to 140°C, and more preferably 115 to 130°C. In the case of other elastomer fibers, the heating temperature for the metal roll may be, for example, [Vicat softening temperature - 50°C] to [Vicat softening temperature + 50°C] based on the Vicat softening temperature of the elastomer fiber constituting the precursor nonwoven fabric, which is measured in accordance with JIS K 7206:2016.

The nonwoven fabric is excellent in adhesion to skin, and can be used as a constituent material of a patch which is applied to skin, such as a medical patch and a wearable device. Examples of the medical patch include wound dressings, adhesive plasters, transdermal patches, adhesive bandages, and fixing materials for medical instruments. Examples of the wearable device include a biomedical sensor that is capable of monitoring biological information such as a body temperature, a blood pressure, a heart rate, and an electrocardiogram. In particular, the nonwoven fabric is excellent in the water resistance, and can be thus used as a backing material of a wound dressing to prevent ingress of moisture and bacteria from the outside.

The medical patch or the wearable device includes the nonwoven fabric described above, and may preferably include the nonwoven fabric as a backing material thereof. Further, the medical patch or the wearable device may at least include: a backing material layer including the nonwoven fabric described above; and a pressure-sensitive adhesive layer including a pressure-sensitive adhesive for using to be applied to skin. Examples of the pressure-sensitive adhesive include natural rubber-based pressure-sensitive adhesives, synthetic rubber-based pressure-sensitive adhesives, acrylic pressure-sensitive adhesives, styreneisoprene (SIS) block copolymer-based pressure-sensitive adhesives, silicone-based pressure-sensitive adhesives, urethane-based pressure-sensitive adhesives, and hydrous pressure-sensitive adhesives. The pressure-sensitive adhesive layer can be formed, for example, by a known method such as a method in which a pressure-sensitive adhesive composition containing a pressure-sensitive adhesive is applied to a backing material or the like and dried, and a method in which a pressure-sensitive adhesive obtained by molding a pressure-sensitive adhesive composition into a predetermined shape is transferred and laminated to a backing material or the like.

The pressure-sensitive adhesive may contain additives such as a tackifier, a filler, a pigment, a pH adjuster, a plasticizer, an antioxidant, and a bioactive component.

A thickness of the pressure-sensitive adhesive layer can be appropriately set depending on the intended use and the like, and may be, for example, about 5 µm to 200 µm from the viewpoint of adhesiveness and adhesion to skin. A basis weight of the pressure-sensitive adhesive layer can be appropriately set depending on the intended use and the like, and may be, for example, about 10 to 100 g/m² from the viewpoint of adhesiveness and flexibility.

An adhesive strength of the pressure-sensitive adhesive can be appropriately set depending on the adherend, the intended use, and the like. For example, an adhesive strength for human skin may be 0.1 to 1.0 N/10 mm from the viewpoint of improving the adhesiveness and reducing the discomfort against the skin. The adhesive strength for human skin is measured by performing a test with the use of the back of human's hand as a pressure-sensitive adhesive target (adherend) after is subjected to degreasing with an isopropyl alcohol and then air drying, with reference to section 10 of JIS Z 0237:2009 "Testing methods of pressure-sensitive adhesive tapes and sheets". Further, for fixing a medical instrument, a sensor, or the like to the skin, the adhesive strength for a SUS plate may be, for example, 0.1 to 10.0 N/10 mm from the viewpoint of improving the adhesiveness to the medical instrument, the sensor, and the like. The adhesive strength for the SUS plate is measured in accordance with section 10 of JIS Z 0237:2009 "Testing methods of pressure-sensitive adhesive tapes and sheets".

A tensile strength of the pressure-sensitive adhesive may be 0.1 to 10.0 N/10 mm from the viewpoint of strength and flexibility as the pressure-sensitive adhesive layer. The tensile strength of the pressure-sensitive adhesive is measured in accordance with section 8 of JIS Z 0237:2009 "Testing methods of pressure-sensitive adhesive tapes and sheets".

A water vapor permeability of the pressure-sensitive adhesive may be 800 to 10,000 g/m²·24h from the viewpoint of suppressing skin stuffiness. The water vapor permeability of the pressure-sensitive adhesive is measured in accordance with section 15 of JIS Z 0237:2009 "Testing methods of pressure-sensitive adhesive tapes and sheets".

The pressure-sensitive adhesive layer may have a single layer structure or a multilayer structure obtained by bonding two or more layers that are different in raw material and/or properties.

The pressure-sensitive adhesive layer may have an adhesive part and a non-adhesive part, and the area of the adhesive part and the uneven structure of the surface can be appropriately designed from the viewpoint of adjusting the air permeability, stretchability, water resistance, and the like of a medical patch or wearable device including the pressure-sensitive adhesive layer. Further, the adhesive part may have a pattern such as a stripe shape, a dot shape, a lattice shape, a rectangular shape, a triangular shape, a polygonal shape, a round shape, an oval shape, and a wave shape.

From the viewpoint of improving the water resistance of the patch, the backing material layer and/or the pressure-sensitive adhesive layer may be subjected to a water repellent treatment. In the water-repellent treatment, the surface of the nonwoven fabric and/or pressure-sensitive adhesive may be coated with a water-repellent material such as a silicone-based resin, a fluorine-based resin, a polystyrene-based resin, and an acrylic resin.

The medical patch or the wearable device may include a release film or a release paper for protecting the pressure-sensitive adhesive layer in addition to the backing material layer including the nonwoven fabric and the pressure-sensitive adhesive layer including the pressure-sensitive adhesive. Examples of the release film or release paper include a polyester film, a polyethylene film, a polypropylene film, a kraft paper, and a glassine paper.

### EXAMPLES

Hereinafter, the present invention will be described in more detail on the basis of Examples, but the present invention is not limited to these Examples whatsoever. In Examples and Comparative Example presented below, various physical properties were measured by the following methods.

### [Thickness, Basis Weight, Bulk Density]

In accordance with section 6.1 of JIS L 1913 "Test methods for nonwovens", a thickness (mm) of each of a precursor nonwoven fabric and a nonwoven fabric was measured using a measuring instrument with a pressing plate with a diameter of 16 mm at a pressing pressure of 10 g/cm².

In accordance with section 6.2 of JIS L 1913 "Test methods for nonwovens", a basis weight (g/m²) of each of the precursor nonwoven fabric and the nonwoven fabric was measured.

A bulk density (g/cm³) of the nonwoven fabric was calculated by dividing the basis weight by the thickness.

### [Air Permeability]

In accordance with section 8.26 of JIS L 1096 "Testing methods for woven and knitted fabrics", an air permeability (cm³/cm²·s) was measured by a Frazier method using an air permeability tester "FX3300" manufactured by TEXTEST AG.

### [Average Fiber Diameter of Elastomer Fiber]

A surface of a precursor nonwoven fabric was observed using a scanning electron microscope. Fiber diameters of 100 single fibers randomly selected from the electron micrograph were measured from side surfaces, and a number average fiber diameter was determined as an average fiber diameter (µm) of elastomer fibers.

### [Water Pressure Resistance]

In accordance with a low water pressure method in JIS L 1092:2009 "Testing methods for water resistance of textiles", a water pressure resistance (mmH₂O) was measured.

### [Stress at 25% Elongation and Elongation]

In accordance with section 6.3 of JIS L 1913 "Test methods for nonwovens", a tensile test was performed. Five test pieces each having a width of 5 cm and a length of 20 cm were prepared, each test piece having a longitudinal direction corresponding to the machine direction (MD) at the time of producing a nonwoven fabric. Using Autograph "AGX-V" manufactured by Shimadzu Corporation, a distance between grips was set to 10 cm, ends of each test piece were then fixed at the grips of the tester, and the test piece was pulled at a speed of 200 mm/min until it was broken. From the obtained stress-strain curve, each of a stress at 25% elongation (N/5 cm) and an elongation at break (%) was determined as an average value (n = 5). In addition, for test pieces each having a longitudinal direction corresponding to the cross direction (CD), the stress (N/5 cm) at 25% elongation and the elongation at break (%) were determined similarly.

### [Bending Resistance]

From a nonwoven fabric, five test pieces each having a long side of 10 cm and a short side of 2 cm were prepared, in which the long side corresponds to an MD and the short side corresponds to a CD. Each of the test pieces was placed on a horizontal surface, the test piece was folded by lifting up one short side end and overlaying it on the other short side end, and the bent part was pressed with a load of 19 kg/m² for 1 second. Thereafter, the curved surface shape part of the test piece, which was formed when the load was removed, was observed from a side surface of the test piece, to measure a distance (mm) from the horizontal surface to the maximum height of the curved surface shaped part. An average value (n = 5) was calculated as the bending resistance (mm).

### [Example 1]

Using a melt-blown nonwoven fabric production apparatus, a polyurethane-based elastomer resin ("KU-490" manufactured by Tosoh Corporation) was spun at a single hole discharge rate of 0.3 g/min·hole (single hole diameter: 300 µm) and a spinning temperature of 240°C, and while blowing hot air at a temperature of 240°C, fibrous discharged materials were scattered onto and collected on a conveyor belt under the condition of collection speed of 14 m/min to obtain a precursor nonwoven fabric shown in Table 1. The obtained precursor nonwoven fabric was sandwiched between a rubber roll and a metal flat roll under the conditions of the heating temperature for the metal flat roll of 126°C and a linear pressure of 50 kg/cm. In such manner, the obtained precursor nonwoven fabric was subjected to calendaring to obtain a nonwoven fabric shown in Table 1.

### [Example 2]

A precursor nonwoven fabric shown in Table 1 was obtained in the same manner as in Example 1 except that in the melt-blowing method, the single hole discharge rate was set to 0.2 g/min·hole, a suction roll with a diameter of 35 cm was used for the collection, and the fibrous discharged materials were collected on the suction roll as shown in Fig. 3, in which the center of the discharge hole and the rotation center of the suction roll were located along the vertical direction, under the condition of the collection speed of 12 m/min. Thereafter, a nonwoven fabric shown in Table 1 was obtained in the same manner as in Example 1 except for calendering under the condition of the heating temperature for the metal flat roll of 120°C.

### [Example 3]

A precursor nonwoven fabric shown in Table 1 was obtained in the same manner as in Example 1 except that in the melt-blowing method, the single hole discharge rate was set to 0.2 g/min·hole, a suction roll with a diameter of 35 cm was used for the collection, and the fibrous discharged materials were collected on the suction roll as shown in Fig. 4, in which the collection position was located at a position of 10 cm from the rotation center in the horizontal direction on the upstream side in the rotating direction of the suction roll, under the condition of the collection speed of 12 m/min. Thereafter, a nonwoven fabric shown in Table 1 was obtained in the same manner as in Example 1 except for calendering under the conditions of the heating temperature for the metal flat roll of 120°C and the linear pressure between the rolls of 50 kg/cm.

### [Example 4]

A precursor nonwoven fabric shown in Table 1 was obtained in the same manner as in Example 1 except that in the melt-blowing method, the single hole discharge rate was set to 0.4 g/min·hole, the spinning temperature was set to 250°C, and the collection speed was set to 11 m/min. Thereafter, a nonwoven fabric shown in Table 1 was obtained in the same manner as in Example 1 except for calendering under the condition of the heating temperature for the metal flat roll of 120°C.

### [Example 5]

Using the melt-blown nonwoven fabric shown in Table 1 as a support, which was prepared in the same manner as in Example 1, a melt-blown nonwoven fabric was formed thereon under the same conditions for the melt blowing method in Example 1 except that the single hole discharge rate was set to 0.5 g/min·hole, the spinning temperature was set to 250°C, and the collection speed was set to 17 m/min, to obtain a precursor layered body shown in Table 1. The obtained precursor layered body was sandwiched between a rubber roll and a metal flat roll under the conditions of the heating temperature for the metal flat roll of 126°C and a linear pressure of 50 kg/cm, in such a way that the melt-blown nonwoven fabric side having a large average fiber diameter came into contact with the rubber roll and the melt-blown nonwoven fabric side having a small average fiber diameter came into contact with the metal flat roll. In such manner, the obtained precursor layered body was subjected to calendaring to obtain a nonwoven fabric shown in Table 1.

### [Example 6]

Using a melt-blown nonwoven fabric shown in Table 1 as a support, which was prepared in the same manner as in Example 2 except that the collection speed was set to 8 m/min, a melt-blown nonwoven fabric was formed thereon under the same conditions for the melt blowing method in Example 5 to obtain a precursor layered body shown in Table 1. The obtained precursor layered body was subjected to calendering under the same conditions in Example 5 to obtain a nonwoven fabric shown in Table 1.

### [Example 7]

Using a melt-blown nonwoven fabric production apparatus, a resin obtained by mixing a styrene-based elastomer resin ("SEPTON 2002" manufactured by Kuraray Co., Ltd.) and a polypropylene resin ("S13BWA" manufactured by Prime Polymer Co., Ltd.) at a weight ratio of 70 : 30 was spun at a single hole discharge rate of 0.3 g/min·hole (single hole diameter: 300 µm) and a spinning temperature of 295°C, and while blowing hot air at a temperature of 295°C, fibrous discharged materials were scattered onto and collected on a conveyor belt under the condition of collection speed of 10 m/min to obtain a precursor nonwoven fabric shown in Table 1. The obtained precursor nonwoven fabric was sandwiched between a rubber roll and a metal flat roll under the conditions of the heating temperature for the metal flat roll of 105°C and a linear pressure of 50 kg/cm. In such manner, the obtained precursor nonwoven fabric was subjected to calendaring to obtain a nonwoven fabric shown in Table 1.

### [Example 8]

A precursor nonwoven fabric shown in Table 1 was obtained in the same manner as in Example 7 except that in the melt-blowing method, the collection speed was set to 15 m/min. The obtained precursor nonwoven fabric was sandwiched between a rubber roll and a metal flat roll under the conditions of the heating temperature for the metal flat roll of 105°C and a linear pressure of 50 kg/cm. In such manner, the obtained precursor nonwoven fabric was subjected to calendaring to obtain a nonwoven fabric shown in Table 1.

### [Comparative Example 1]

A precursor nonwoven fabric shown in Table 1 was obtained in the same manner as in Example 1 except that in the melt-blowing method, the single hole discharge rate was set to 0.5 g/min·hole, the spinning temperature was set to 250°C, and the collection speed was set to 4 m/min. Thereafter, a nonwoven fabric shown in Table 1 was obtained in the same manner as in Example 1 except for calendering under the condition of the heating temperature for the metal flat roll of 120°C.

### [Comparative Example 2]

A precursor nonwoven fabric shown in Table 1 was obtained in the same manner as in Example 1 except that in the melt-blowing method, the single hole discharge rate was set to 0.2 g/min·hole and the collection speed was set to 12 m/min. Thereafter, a nonwoven fabric shown in Table 1 was obtained in the same manner as in Example 1 except for using a metal embossing roll instead of the metal flat roll and calendering under the condition of the heating temperature for the metal embossing roll of 120°C.

### [Comparative Example 3]

A precursor nonwoven fabric shown in Table 1 was obtained in the same manner as in Example 1 except that in the melt-blowing method, the single hole discharge rate was set to 0.2 g/min·hole and the collection speed was set to 12 m/min. Thereafter, a nonwoven fabric shown in Table 1 was obtained in the same manner as in Example 1 except for calendering under the condition of the heating temperature for the metal flat roll of 100°C.

### [Comparative Example 4]

As Comparative Example 4, a urethane-based film ("OFU-6030B" manufactured by Ohishi Sangyo Co., Ltd.) shown in Table 1 was prepared and evaluated in the same manner as for the nonwoven fabric as shown in Table 1. Since Comparative Example 4 was the film, the air permeability was not more than the measurement limit and the water pressure resistance was not less than the measurement limit, and therefore, these measurement values could not be detected.

### [Comparative Example 5]

A precursor nonwoven fabric shown in Table 1 was obtained in the same manner as in Example 1 except that in the melt-blowing method, the single hole discharge rate was set to 0.2 g/min·hole and the collection speed was set to 12 m/min. Thereafter, a nonwoven fabric shown in Table 1 was obtained in the same manner as in Example 1 except for calendering under the condition of the heating temperature for the metal flat roll of 105°C.

### [Comparative Example 6]

A precursor nonwoven fabric shown in Table 1 was obtained in the same manner as in Example 1 except that in the melt-blowing method, the single hole discharge rate was set to 0.4 g/min·hole, the spinning temperature was set to 245°C, and the collection speed was set to 24 m/min. Thereafter, a nonwoven fabric shown in Table 1 was obtained in the same manner as in Example 1 except for calendering under the condition of the heating temperature for the metal flat roll of 122°C.

**[Table 1]**

| | | | | Ex. 1 | Ex. 2 | Ex. 3 | Ex. 4 | Ex. 5 | Ex. 6 | Ex. 7 | Ex. 8 |
|---|---|---|---|---|---|---|---|---|---|---|---|
| Producing method | Elastomer type | | - | Urethane-based | Urethane-based | Urethane-based | Urethane-based | Urethane-based/ urethane-based | Urethane-based/ urethane-based | Styrene-based/PP (70/30) | Styrene-based/PP (70/30) |
| | Melt-blowing method | Collection method | - | Conveyor belt | Rotating roll (Fig. 3) | Rotating roll (Fig. 4) | Conveyor belt | Conveyor belt | Rotating roll (Fig. 3)/ conveyor belt | Conveyor belt | Conveyor belt |
| | | Spinning temperature | °C | 240 | 240 | 240 | 250 | 240/250 | 240/250 | 295 | 295 |
| | | Single hole discharge rate | g/min·H | 0.3 | 0.2 | 0.2 | 0.4 | 0.3/0.5 | 0.2/0.5 | 0.3 | 0.3 |
| | | Collection speed | m/min | 14 | 12 | 12 | 11 | 14/17 | 8/17 | 10 | 15 |
| | Pressing | Pressing roll | - | Flat | Flat | Flat | Flat | Flat | Flat | Flat | Flat |
| | | Pressing temperature | °C | 126 | 120 | 120 | 120 | 126 | 126 | 105 | 105 |
| | | Pressing pressure | kg/cm | 50 | 50 | 50 | 50 | 50 | 50 | 50 | 50 |
| Precursor nonwoven fabric | Thickness | | µm | 132 | 95 | 78 | 307 | 132/215 | 45/215 | 203 | 176 |
| | Basis weight | | g/m² | 29.4 | 21.9 | 22.1 | 50.1 | 30.0/40.0 | 30.0/40.0 | 31.3 | 21.0 |
| | Average fiber diameter | | µm | 13 | 4 | 3 | 13 | 13/15 | 6/15 | 5 | 5 |
| Nonwoven fabric | Thickness | | µm | 52 | 44 | 36 | 104 | 130 | 125 | 60 | 38 |
| | Basis weight | | g/m² | 29.4 | 21.9 | 22.1 | 50.1 | 70.0 | 70.0 | 30.9 | 20.0 |
| | Bulk density | | g/cm³ | 0.57 | 0.50 | 0.61 | 0.48 | 0.54 | 0.56 | 0.52 | 0.53 |
| | Air permeability | | cm³/cm²·s | 0.2 | 1.2 | 0.2 | 3.3 | 0.2 | 0.3 | 0.6 | 0.9 |
| | Air permeability/ basis weight | | - | 0.0068 | 0.0548 | 0.0090 | 0.0659 | 0.0029 | 0.0043 | 0.0201 | 0.0465 |
| | Water pressure resistance | | mmH₂O | 500 | 250 | 300 | 200 | 700 | 1000 | 700 | 450 |
| | Stress at 25% elongation | MD | N/5 cm | 6.1 | 3.7 | 4.8 | 6.2 | 10.0 | 9.2 | 8.2 | 5.5 |
| | | CD | N/5 cm | 5.2 | 2.9 | 3.3 | 5.6 | 8.0 | 8.1 | 6.1 | 4.1 |
| | Elongation | MD | % | 420 | 291 | 309 | 396 | 387 | 380 | 324 | 330 |
| | | CD | % | 298 | 293 | 313 | 345 | 354 | 353 | 212 | 220 |
| | Bending resistance | | mm | 5 | 5 | 4 | 7 | 8 | 8 | 9 | 7 |

**[Table 2]**

| | | | | Com. Ex. 1 | Com. Ex. 2 | Com. Ex. 3 | Com. Ex. 4 | Com. Ex. 5 | Com. Ex. 6 |
|---|---|---|---|---|---|---|---|---|---|
| Producing method | Elastomer type | | - | Urethane-based | Urethane-based | Urethane-based | Urethane-based film | Urethane-based | Urethane-based |
| | Melt-blowing method | Collection method | - | Conveyor belt | Conveyor belt | Conveyor belt | - | Conveyor belt | Conveyor belt |
| | | Spinning temperature | °C | 250 | 240 | 240 | - | 240 | 245 |
| | | Single hole discharge rate | g/min·H | 0.5 | 0.2 | 0.2 | - | 0.2 | 0.4 |
| | | Collection speed | m/min | 4 | 12 | 12 | - | 12 | 24 |
| | Pressing | Pressing roll | - | Flat | Embossing | Flat | - | Flat | Flat |
| | | Pressing temperature | °C | 120 | 120 | 100 | - | 105 | 122 |
| | | Pressing pressure | kg/cm | 50 | 50 | 50 | - | 50 | 50 |
| Precursor nonwoven fabric | Thickness | | µm | 495 | 103 | 110 | - | 110 | 150 |
| | Basis weight | | g/m² | 148 | 20.6 | 20.4 | - | 21.5 | 20.2 |
| | Average fiber diameter | | µm | 17 | 4 | 7 | - | 5 | 9 |
| Nonwoven fabric | Thickness | | µm | 366 | 98 | 93 | 35 | 55 | 40 |
| | Basis weight | | g/m² | 148 | 20.6 | 20.4 | 35.7 | 21.3 | 19.7 |
| | Bulk density | | g/cm³ | 0.40 | 0.21 | 0.22 | 1.02 | 0.39 | 0.49 |
| | Air permeability | | cm³/cm²·s | 3.9 | 41.3 | 50.1 | - | 7.8 | 23.0 |
| | Air permeability/ basis weight | | - | 0.0264 | 2.0049 | 2.4559 | - | 0.3662 | 1.1675 |
| | Water pressure resistance | | mmH₂O | 590 | 120 | 150 | - (1500 or more) | 190 | 90 |
| | Stress at 25% elongation | MD | N/5 cm | 13.0 | 2.8 | 1.9 | 3.7 | 2.9 | 2.8 |
| | | CD | N/5 cm | 10.0 | 2.0 | 1.6 | 3.8 | 2.1 | 2.5 |
| | Elongation | MD | % | 450 | 373 | 407 | 701 | 406 | 265 |
| | | CD | % | 598 | 395 | 424 | 678 | 373 | 258 |
| | Bending resistance | | mm | 22 | 6 | 6 | 1 | 6 | 5 |

As shown in Table 1, the nonwoven fabrics according to Examples 1 to 8 are small in thickness in a specific range, and thus, when applied to skin, are capable of reducing the difference in level between the skin and the patch, and excellent in adhesion to the skin. In addition, the nonwoven fabrics are low in the ratio between the air permeability and the basis weight in a specific range and high in bulk density in a specific range, and are thus excellent in water resistance.

In contrast, as shown in Table 2, the nonwoven fabric according to Comparative Example 1 is relatively high in water pressure resistance, but it is because of the larger thickness of the nonwoven fabric. When the nonwoven fabric having such a larger thickness is applied to skin, the difference in level between the skin and the patch is increased, thereby leading to inferior adhesion to the skin.

The nonwoven fabrics according to Comparative Examples 2 and 3 are small in thickness, but inferior in water resistance because the ratio of air permeability to the basis weight and the bulk density fall outside the specific ranges.

Since Comparative Example 4 is the film, it is low in bending resistance and inferior in self-supporting property.

The nonwoven fabric according to Comparative Example 5 is small in thickness and low in the ratio of the air permeability to the basis weight, but is inferior in water resistance because of the low bulk density.

The nonwoven fabric according to Comparative Example 6 is small in thickness and high in bulk density, but is inferior in water resistance because of the high ratio of the air permeability to the basis weight.

### INDUSTRIAL APPLICABILITY

The nonwoven fabric can be suitably used as a constituent material in a medical patch, a wearable device, or the like which is used by applying it to skin.

Although the present invention has been described in connection with the preferred embodiments thereof with reference to the accompanying drawings, those skilled in the art will readily conceive numerous changes and modifications within the framework of obviousness upon the reading of the specification presented herein. Accordingly, such changes and modifications are construed as within the scope of the invention as defined by the scope of the claims.

### REFERENCE NUMERALS

- 10: Nonwoven fabric
- 11: Long side end
- 12: Short side end
- 13: Bent part
- 14: Curved surface shaped part
- 21, 31, 41: Spinneret
- 22, 32, 42: Discharged material
- 23, 33, 43: Collection surface
- 24: Conveyor belt
- 35, 45: Rotating roll

## Claims

1. A nonwoven fabric comprising an elastomer fiber, wherein the nonwoven fabric has a thickness of 200 µm or less, a ratio (air permeability/basis weight) of an air permeability (cm³/cm²·s) to a basis weight (g/m²) of 1.0 or less, and a bulk density of 0.47 g/cm³ or more.

2. The nonwoven fabric according to claim 1, having the basis weight of 10 to 100 g/m².

3. The nonwoven fabric according to claim 1 or 2, wherein the elastomer fiber is a urethane-based elastomer fiber.

4. The nonwoven fabric according to claim 1 or 2, having a stress at 25% elongation of 10 N/5 cm or less in at least one direction in a plane direction.

5. The nonwoven fabric according to claim 1 or 2, having an elongation of 200% or more in at least one direction in a plane direction.

6. The nonwoven fabric according to claim 1 or 2, having a water pressure resistance of 170 mmH₂O or more measured in accordance with a low water pressure method in JIS L 1092:2009.

7. The nonwoven fabric according to claim 1 or 2, wherein the nonwoven fabric is a layered body comprising nonwoven fabrics that are different in average fiber diameter.

8. A medical patch comprising the nonwoven fabric according to claim 1 or 2.

9. The medical patch according to claim 8, comprising a pressure-sensitive adhesive layer.

10. A wearable device comprising the nonwoven fabric according to claim 1 or 2.

11. The wearable device according to claim 10, comprising a pressure-sensitive adhesive layer.
